# EUROPÄISCHE PATENTSCHRIFT

(11) **EP 3 022 075 B1**
(45) Veröffentlichungstag und Bekanntmachung des Hinweises auf die Patenterteilung: **01.03.2017**
(21) Anmeldenummer: 14739197.3
(22) Anmeldetag: 16.07.2014
(51) Int. Cl.: B60H 3/00, A61L 9/12

(54) **ANSCHLUSSVORRICHTUNG**
CONNECTING APPARATUS
DISPOSITIF DE RACCORDEMENT

(30) Priorität: 19.07.2013 DE 102013012021
(43) Veröffentlichungstag der Anmeldung: 25.05.2016
(73) Patentinhaber: Leopold Kostal GmbH & Co. KG, 58513 Lüdenscheid (DE)
(72) Erfinder: HAFNER, Tobias, 44139 Dortmund (DE)
(74) Vertreter: Kerkmann, Detlef
(86) Internationale Anmeldenummer: PCT/EP2014/065295
(87) Internationale Veröffentlichungsnummer: WO 2015/007794

(56) Entgegenhaltungen:
- DE-A1- 10 344 308
- DE-A1-102006 028 399
- DE-A1-102010 008 436

## Beschreibung

Die vorliegende Erfindung betrifft eine Anschlussvorrichtung zur Anbindung eines Duftstoffbehälters an ein elektrisches Gerät zur Duftausbringung in einem Kraftfahrzeug, mit einem Duftstoffbehälter, der eine Anschlussfläche mit einer federbelasteten Ventilplatte aufweist, und mit einem am Gerät angeordneten Anschlussadapter, der am angefügten Duftstoffbehälter die Ventilplatte öffnet.

Aus der deutschen Offenlegungsschrift DE 10 2010 008 436 A1 ist ein Gerät zur Duftausbringung in einem Kraftfahrzeug bekannt, bei dem ein Duftstoffbehälter eine Ventilkappe mit einer federbelasteten Ventilplatte aufweist, die beim Anfügen an einen Anschlussadapter in eine Öffnungsstellung verschoben wird. Durch den unbeweglich am Gerät angeordneten Anschlussadapter wird der Duftstoffbehälter strömungsleitend mit einer elektrischen Einrichtung zur Erzeugung eines Luftstroms verbunden, die den in dem Duftstoffbehälter enthaltenen Duftstoff dosiert im Innenraum des Kraftfahrzeugs verteilt.

Die Ventilplatte ist mit einer durch den Hals des Duftstoffbehälters geführten rohrförmigen Anformung verbunden, welche einen Bestandteil der Ventil mechanik ausbildet. Hierdurch ist die Ventilplatte zwar senkrecht zu ihrer Hauptfläche verschiebbar, aber nur geringfügig verkippbar. Eine Verkippung des Duftstoffbehälters gegen den unbeweglich am Gerät angeordneten Anschlussadapter kann daher in nur geringem Maß durch die Ventilplatte ausgeglichen werden.

Bei einem Gerät zur Duftstoffausbringung ist sicherzustellen, dass nach dem Verbinden von Gerät und Duftstoffbehälter zwischen diesen eine gasdichte Verbindung besteht, damit der mit dem Duftstoff angereicherte Luftstrom durch das Gesamtsystem bis zum Zielort geleitet wird und nicht an unerwünschter Stelle austritt. Beim Verbinden entstehen durch das Zusammenspiel unterschiedlicher Bauteile nicht nur Toleranzen in der Höhe, sondern auch Winkelfehler, welche ausgeglichen werden müssen. Da die Behälteroberfläche eine Ebene ist, die in mehrere Richtungen gekippt sein kann, beschränkt sich der Winkelfehler nicht nur auf eine Richtungsachse.

Es stellte sich daher die Aufgabe, eine Anschlussvorrichtung zu schaffen, welche einen Winkelversatz an der Verbindungsstelle zwischen Gerät und Duftstoffbehälter selbsttätig ausgleicht.

Diese Aufgabe wird erfindungsgemäß dadurch gelöst, dass der Anschlussadapter ein erstes, relativ zum Gerät feststehenden Bauteil aufweist, und dass der Anschlussadapter ein zweites Bauteil aufweist, das gegen das erste Bauteil gegen die Kraft mindestens einer Feder verschwenkbar ist und an die Anschlussfläche des Duftstoffbehälters anfügbar ist.

Erfindungsgemäß ist vorgesehen, dass der Anschlussadapter ein erstes feststehendes und ein zweites, um die Hochachse schwenkbares Bauteil aufweist. Beide Bauteile sind durch mindestens ein toleranzausgleichendes elastisches oder federndes Element, zum Beispiel durch eine oder mehrere Schraubenfeder, miteinander verbunden.

Vorteilhafte Ausgestaltungen und Weiterbildungen ergeben sich aus der folgenden Erläuterung eines Ausführungsbeispiels der Erfindung anhand der Zeichnung. Es zeigen
- Figur 1: einen Anschlussadapter in einer Draufsicht,
- Figur 2: den Anschlussadapter in einer Explosionsansicht,
- Figuren 3a, 3b: zwei skizzenhafte Darstellungen jeweils einer mit einem Duftstoffbehälter verbundenen Anschlussvorrichtung,
- Figur 4: den Anschlussadapters in einer Schnittansicht,
- Figur 5: eine Draufsicht auf die Anschlussfläche des Duftstoffbehälters.

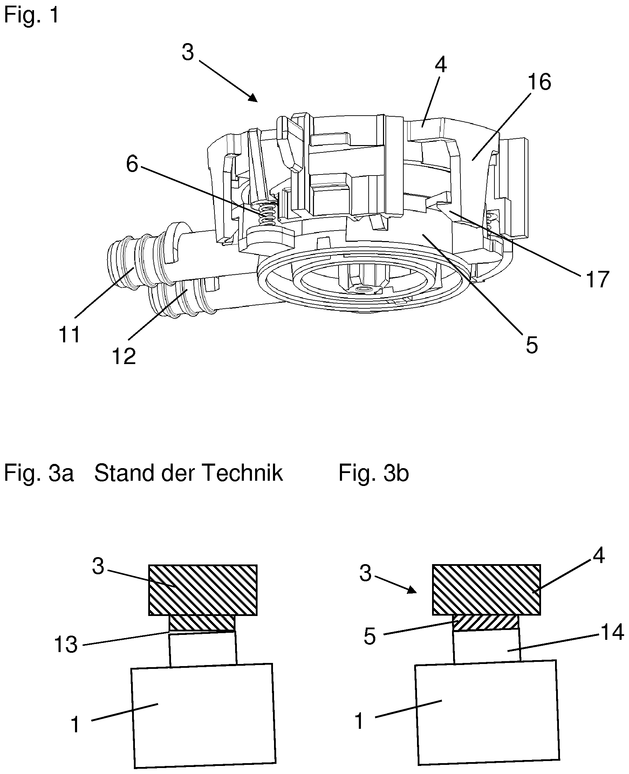

Zunächst soll anhand der Figur 3a das zur Erfindung führende Problem und in der Figur 3b das vorgeschlagene Lösungsprinzip kurz erläutert werden. Skizziert ist jeweils ein Duftstoffbehälter 1, der mit einem Anschlussadapter 3 verbunden ist. Der Anschlussadapter 3 ist jeweils ein Bestandteil eines hier nicht dargestellten elektrischen Gerätes, welches einen Duftstoff in einem Kraftfahrzeug ausbringen kann. Zur genauen Ausgestaltung eines solches Geräts sei auf die deutsche Offenlegungsschrift DE 10 2010 008 436 A1 verwiesen, welche Aufbau und Funktion ausführlich beschreibt. Prinzipiell besteht die Funktion des Gerätes darin, einen Luftstrom zu erzeugen, der dampfförmigen Duftstoff aus dem Duftstoffbehälter fördert und über Rohr- oder Schlauchverbindungen an bestimmte Stellen des Kraftfahrzeugs transportiert.

Da der Duftstoffbehälter 1 kein fester Bestandteil des elektrischen Gerätes ist, sondern an das Gerät angefügt und auch von diesem getrennt werden kann, ist der Anschlussadapter 3 vorgesehen, welcher den Innenraum des Duftstoffbehälters 1 strömungsleitend mit dem elektrischen Gerät verbindet. Dabei ist sicherzustellen, dass die Anbindung zwischen dem Duftstoffbehälter 1 und dem Anschlussadapter 3 gasdicht ist, damit an der Verbindungsstelle keine Dämpfe des Duftstoffs unerwünschterweise austreten können.

Da zwischen dem Duftstoffbehälter 1 und dem Anschlussadapter 3 absichtlich keine formschlüssige Verbindung, beispielsweise über einen Schraubanschluss, vorgesehen ist, um ein besonders einfaches Verbinden und Trennen zu ermöglichen, ergibt sich das Problem, dass eine ungenaue kraftschlüssige Verbindung zwischen dem Duftstoffbehälter 1 und dem Anschlussadapter 3 schnell zu Undichtigkeiten führen kann.

Ist etwa der Duftstoffbehälter 1 nur leicht gegen den unbeweglich angeordneten Anschlussadapter 3 verkippt, so ergibt sich, wie die Figur 3a andeutet, an der Verbindungsstelle ein Zwischenraum 13, aus dem dampfförmiger Duftstoff entweichen kann.

Die in der Figur 3b skizzierte Lösung dieses Problems besteht darin, dass der Anschlussadapter 3 ein erstes, relativ zu den hier nicht dargestellten übrigen Komponenten des Geräts feststehendes erstes Bauteil 4, und ein relativ zu diesem ersten Bauteil 4 verschwenkbar angeordnetes zweites Bauteil 5 aufweist, welches Lagetoleranzen des Duftstoffbehälters 1 ausgleicht. Wie die Figur 3b zeigt, ist das zweite Bauteil 5 gegenüber dem feststehenden Bauteil 4 verschwenkt und liegt dadurch plan an der Oberseite des Duftstoffbehälterhalses 14 an.

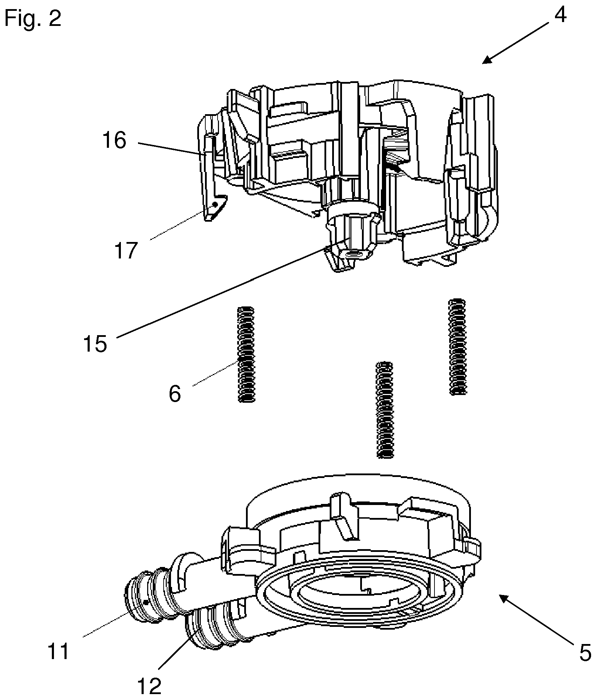

Ein Konstruktionsbeispiel eines Anschlussadapters 3 ist in den Figuren 1 und 2 dargestellt. Die Figur 1 zeigt einen Anschlussadapter 3, der aus einem ersten Bauteil 4 und einem daran gelagerten zweiten Bauteil 5 zusammengefügt ist, zwischen denen eine Schraubenfeder 6 erkennbar ist.

Die Figur 2 verdeutlicht die Komponenten des Anschlussadapters 3 in einer Explosionsdarstellung. Das erste Bauteil 4, welches an einem hier nicht dargestellten Gerät fest angebracht ist, weist eine zentrale stiftförmige Anformung 15 auf, um die herum, in einer symmetrischen Anordnung drei Schraubenfedern 6 angeordnet sind. Die Schraubenfedern 6 stützen sich außerdem an dem zweiten Bauteil 5 ab, welches durch mehrere Halteelemente 16 am ersten Bauteil 4 gelagert ist. Wie aus der Figur 1 hervorgeht, pressen die Schraubenfedern 6 das Gehäuse des zweiten Bauteils gegen Anlageflächen an hakenförmigen Endabschnitten 17 der Halteelemente 16.

Durch eine von unten auf das zweite Bauteil 5 einwirkende Kraft können die Schraubenfeder 6 zusammengedrückt, so dass das zweite Bauteil 5 gegen das erste Bauteilteil 4 axial verschoben oder auch um zwei Richtungsachsen in der horizontalen Ebene verschwenkt werden kann. Hierdurch können beliebige Fehlorientierungen des Duftstoffbehälters 1 gegen den Anschlussadapter 3 ausgeglichen werden.

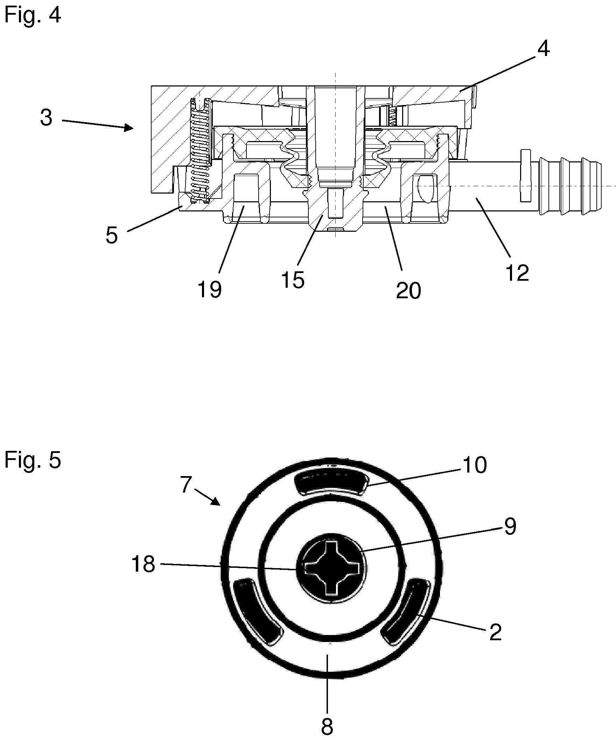

Die strömungsleitende Verbindung zwischen dem Anschlussadapter 3 und der Anschlussfläche 8 des Duftstoffbehälters 1, die sich vorzugsweise an der Oberseite einer an den Duftstoffbehälter 1 anschraubbaren Ventilkappe 7 befindet, wird im Folgenden durch die Figuren 4 und 5 erläutert. Die Figur 5 zeigt die Oberseite der Ventilkappe 7 eines Duftstoffbehälters 1. Die Ventilkappe 7 weist eine zentrale Ventilöffnung 9 und mehrere, hier beispielhaft drei, ringabschnittsförmige Ventilöffnungen 10 auf. Diese Ventilöffnungen 9, 10 werden durch eine innerhalb der Ventilkappe 7 angeordnete federbelastete Ventilplatte 2 verschlossen. Die die Ventilöffnungen 9, 10 verschließende Abschnitte der Ventilplatte 2 sind in der Figur 5 durch dunkle Flächen dargestellt. Durch eine Druckbetätigung eines mit der Ventilplatte 2 verbundenen Ventilstößel 18 wird die Ventilplatte 2 gegen die Kraft einer im Inneren der Ventilkappe 7 angeordneten, hier nicht dargestellten Ventilfeder verschoben, wodurch die Ventilöffnungen 9, 10 freigegeben werden.

Diese Druckbetätigung erfolgt beim Anfügen des Duftstoffbehälters 1 an den Anschlussadapter 3, und zwar durch die stiftförmige Anformung 15 des Anschlussadapters 3, der auf den Ventilstößel 18 einwirkt. Dabei greift die stiftförmige Anformung 15 in die zentrale Ventilöffnung 9 ein, was zugleich sicherstellt, dass die Anschlussfläche 8 des Duftstoffbehälters ausreichend genau zentriert unterhalb des Anschlussadapters 3 angeordnet ist. Hierdurch befinden sich die ringabschnittsförmigen Ventilöffnungen 10 genau unterhalb des in der Figur 4 im Schnitt dargestellten Ringspaltes 19, welche strömungsleitend mit einem Einlasskanal 11, der in den Figuren 1 und 2 erkennbar ist, verbunden ist. Entsprechend wird die zentrale Ventilöffnung 9 von einer die stiftförmige Anformung 15 umgebenden Kammer 20 umfasst, welche strömungsleitend mit einem Auslasskanal 12 verbunden ist.

Das gegen das feststehende erste Bauteil 4 verschwenkbare zweite Bauteil 5 legt sich beim Anfügen des Duftstoffbehälters, unterstützt durch die Druckkraft der Schraubenfedern 6, eng an die Anschlussfläche 8 des Duftstoffbehälters 1 an, wodurch jegliche Lagetoleranzen des Duftstoffbehälters 1 ausgeglichen werden.

### Bezugszeichen

- 1: Duftstoffbehälter
- 2: Ventilplatte
- 3: Anschlussadapter
- 4: erstes (feststehendes) Bauteil
- 5: zweites (verschwenkbares) Bauteil
- 6: Feder (Schraubenfedern)
- 7: Ventilkappe
- 8: Anschlussfläche
- 9: zentrale Ventilöffnung
- 10: ringabschnittsförmige Ventilöffnung
- 11: Einlasskanal (Anschlusskanal)
- 12: Auslasskanal (Anschlusskanal)
- 13: Zwischenraum
- 14: Duftstoffbehälterhals
- 15: stiftförmige Anformung
- 16: Halteelemente
- 17: hakenförmige Endabschnitte
- 18: Ventilstößel
- 19: Ringspalt
- 20: Kammer

## Patentansprüche

1. Anschlussvorrichtung zur Anbindung eines Duftstoffbehälters (1) an ein elektrisches Gerät zur Duftausbringung in einem Kraftfahrzeug,
mit einem Duftstoffbehälter (1), der eine Anschlussfläche (8) mit einer federbelasteten Ventilplatte (2) aufweist, und
mit einem am Gerät angeordneten Anschlussadapter (3), der am angefügten Duftstoffbehälter (1) die Ventilplatte (2) öffnet,
**dadurch gekennzeichnet,**
**dass** der Anschlussadapter (3) ein erstes, relativ zum Gerät feststehenden Bauteil (4) aufweist, und
**dass** der Anschlussadapter (3) ein zweites Bauteil (5) aufweist, das gegen das erste Bauteil (4) gegen die Kraft mindestens einer Feder (6) verschwenkbar ist und an die Anschlussfläche (8) des Duftstoffbehälters (1) anfügbar ist.

2. Anschlussvorrichtung nach Anspruch 1, **dadurch gekennzeichnet, dass** die Anschlussfläche (8) Bestandteil einer am Duftstoffbehälter (1) befestigbaren Ventilkappe (7) ist.

3. Anschlussvorrichtung nach Anspruch 1, **dadurch gekennzeichnet, dass** die Anschlussfläche (8) eine zentrale Ventilöffnung (9) und mindestens eine weitere Ventilöffnung (10) aufweist.

4. Anschlussvorrichtung nach Anspruch 1, **dadurch gekennzeichnet, dass** das zweite Bauteil eine Kammer (20) ausbildet, die mit einem Anschlusskanal (12) strömungsleitend verbunden ist, und die von einem Ringspalt (19) umgeben ist, der mit einem weiteren Anschlusskanal (11) strömungsleitend verbunden ist.

## Claims

1. Connection apparatus for affixing a fragrance container (1) to an electrical device for the purpose of discharging fragrance in a motor vehicle,
having a fragrance container (1) which has a connection face (8) with a springloaded valve plate (2), and
having a connecting adapter (3) on the device which opens the valve plate (2) on the attached fragrance container (1),
**characterised in that**
the connecting adapter (3) has a first component (4) which is stationary in relation to the device, and
that the connecting adapter (3) has a second component (5) which can be swivelled against the first component (4) against the force of at least one spring (6) and can be joined to the connection face (8) of the fragrance container (1).

2. Connection apparatus according to Claim 1, **characterised in that** the connecting surface (8) is a constituent of a valve cap (7) that can be attached to the fragrance container (1).

3. Connection apparatus according to Claim 1, **characterised in that** the connecting surface (8) has one central valve opening (9) and at least one further valve opening (10).

4. Connection apparatus according to Claim 1, **characterised in that** the second component constitutes a chamber (20) which is connected in a flow guiding manner to a connecting channel (12) and which is enclosed by an annular gap (19) which is connected in a flow guiding manner to a further connecting channel (11).

## Revendications

1. Dispositif de raccordement pour relier un conteneur de substance odoriférante (1) à un appareil électrique pour en répandre l'odeur dans un véhicule automobile,
avec un conteneur de substance odoriférante (1), qui présente une surface de raccordement (8) pourvue d'une plaque de soupape (2) commandée par ressort, et
avec un adaptateur (3) de raccordement, qui, disposé sur l'appareil, ouvre la plaque de soupape (2) sur le conteneur de substance odoriférante (1) raccordé,
**caractérisé en ce que**
l'adaptateur de raccordement (3) comprend un premier composant (4), qui est fixe par rapport à l'appareil, et
l'adaptateur de raccordement (3) comprend un deuxième composant (5) qui peut être pivoté contre le premier composant (4) contre la force d'au moins un ressort (6) et peut être ajouté à la surface de raccordement (8) du conteneur de substance odoriférante (1).

2. Dispositif de raccordement selon la revendication 1, **caractérisé en ce que** la surface de raccordement (8) fait partie d'une plaque de soupape (7) qui peut être fixée sur le conteneur de substance odoriférante (1).

3. Dispositif de raccordement selon la revendication 1, **caractérisé en ce que** la surface de raccordement (8) présente une ouverture de soupape centrale (9) et au moins une autre ouverture de soupape (10)

4. Dispositif de raccordement selon la revendication 1, **caractérisé en ce que** le deuxième composant forme une chambre (20), qui est reliée à un canal de raccordement (12) conduisant le flux, et qui est entourée d'une fente annulaire (19) qui est reliée à un autre canal de raccordement (11) conduisant le flux.
